# EUROPÄISCHE PATENTSCHRIFT

(11) **EP 2 385 852 B1**
(45) Veröffentlichungstag und Bekanntmachung des Hinweises auf die Patenterteilung: **17.02.2016**
(21) Anmeldenummer: 09797053.7
(22) Anmeldetag: 28.12.2009
(51) Int. Cl.: A61M 15/00, G01F 11/24

(54) **DOSIERVORRICHTUNG**
DOSING DEVICE
DISPOSITIF DE DOSAGE

(30) Priorität: 30.12.2008 DE 202008017185 U
(43) Veröffentlichungstag der Anmeldung: 16.11.2011
(73) Patentinhaber: Sanofi SA, 1214 Vernier (CH)
(72) Erfinder: VON SCHUCKMANN, Alfred, 47627 Kevelaer (DE)
(74) Vertreter: Epping - Hermann - Fischer
(86) Internationale Anmeldenummer: PCT/EP2009/067948
(87) Internationale Veröffentlichungsnummer: WO 2010/076302

(56) Entgegenhaltungen:
- EP-A2- 1 905 472
- EP-A2- 1 905 473
- WO-A1-2005/102430
- US-A- 5 320 714
- US-A- 6 149 054

## Beschreibung

Die Erfindung betrifft eine Dosiervorrichtung zur Inhalierung einer pulverförmigen Substanz, insbesondere medizinischer Art, die in einer Vorratskammer oberhalb eines nachlaufenden Bodens angeordnet und aus dieser in eine Entleerungsbereitschaftsstellung bringbar ist, und mit einer dem echten Füllstand zugeordneten Anzeige im Bereich der Vorrichtungswand.

Dosiervorrichtungen der in Rede stehenden Art dienen der dosierten Inhalation einer vorbestimmten Teilmenge der in der Vorratskammer aufgenommen pulverförmigen Substanz, weiter zur oralen und/ oder nasalen Inhalation. Vorbereitend, gegebenenfalls im Zuge der Inhalation wird hierzu aus der Vorratskammer eine vorbestimmte Teilmenge entnommen und diese in eine Entleerungsbereitschaftsstellung verbracht, aus welcher heraus die abgeteilte Menge im Zuge der Inhalation in den Saugluftstrom überführt wird. Entsprechend der entnommenen Pulvermenge läuft der Boden der Vorratskammer nach der Pulverentnahme nach, dies gegebenenfalls federunterstützt. Entsprechend läuft der Boden in Abhängigkeit von den Inhalationsvorgängen sukzessive in Richtung auf die Vorratskammerdecke unter Verringerung des Kammerfüllvolumens. Um dem Benutzer eine Information über die Anzahl der bereits durchgeführten bzw. über die Anzahl der noch verbleibenden Inhalationsvorgänge zu bieten, ist es weiter bekannt eine dem echten Füllstand zugeordnete Anzeige vorzusehen.

Dosiervorrichtungen sind zum Beispiel aus US 5,320,714 oder EP 1905 472 A2 bekannt.

Im Hinblick auf den bekannten Stand der Technik wird eine technische Problematik der Erfindung darin gesehen, eine Dosiervorrichtung der in Rede stehenden Art insbesondere hinsichtlich einer verbesserten Anzeige in vorteilhafter Weise weiterzubilden.

Diese Problematik ist zunächst und im Wesentlichen durch den Gegenstand des Anspruches 1 gelöst, wobei darauf abgestellt ist, dass die Anzeige aus einem vom Boden mitgeschleppten, um 180° umgelenkten Streifenband besteht, wobei der Umlenkbereich des Streifenbandes hinter der durchsichtigen Vorrichtungswand liegt. Zufolge dieser Ausgestaltung ist eine verbesserte Anzeige, insbesondere eine für den Benutzer verbessert ablesbare Anzeige geschaffen. Die zur Vorbereitung eines Inhalationsvorganges entnommene Pulvermenge aus der Vorratskammer ist in der Regel so gering bemessen, dass eine entsprechend geringe Boden-Nachlaufbewegung hieraus resultiert. Eine unmittelbare Anzeige, abgeleitet aus der Bodenbewegung ist zufolge der vorbeschriebenen Problematik kaum oder nur schwer abzulesen. Es ist hierbei kein merklicher Unterschied der Anzeigestellung gegenüber der vorherigen Stellung vor dem durchgeführten Inhalationsvorgang erkennbar. Durch die Anordnung des vom Boden mitgeschleppten und um 180° umgelenkten Streifenbandes ist eine Übersetzung erreicht, so dass im Bereich der Anzeige bei einfacher 180°-Umkehrung eine Verdopplung des Streifenbandverlagerungsweges gegenüber dem Bodenverlagerungsweg durchgeführt wird. Die Anzeigeverlagerung ist entsprechend für den Benutzer besser erkennbar. Der Umlenkbereich des mitgeschleppten Streifenbandes ist zudem der für den Benutzer durch die durchsichtige Vorrichtungswand visuell erfassbare Anzeigebereich für den Ist-Zustand. In bevorzugter Ausgestaltung ist eine 180°-Umlenkung des Streifenbandes vorgesehen zur entsprechenden 2:1-Übersetzung des Bodenverlagerungsweges. In weiterer Ausgestaltung können auch zwei oder mehr 180°-Umlenkungen vorgesehen sein, zur entsprechenden weiteren Erhöhung des Übersetzungsverhältnisses und daraus resultierender erhöhter Sensibilisierung der Anzeige. Weiter ist das Streifenband mit einer zum Sichtfenster bzw. zur durchsichtigen Vorrichtungswand gewandten Markierungen und/oder einem Fahrverlauf versehen, wobei die Markierung bzw. die Farbe im Bereich der Umlenkung dem Anzeigewert entspricht, entsprechend weiter die Anzahl der noch durchführbaren Inhalationsvorgänge oder alternativ die Anzahl der bereits durchgeführten Inhalationsvorgänge darstellend.

Weitere Merkmale der Erfindung sind nachstehend, auch in der Figurenbeschreibung, oftmals in ihrer bevorzugten Zuordnung zum Gegenstand des Anspruches 1 oder zu Merkmalen weiterer Ansprüche erläutert. Sie können aber auch in einer Zuordnung zu nur einzelnen Merkmalen des Anspruches 1 oder des jeweiligen weiteren Anspruches oder jeweils unabhängig von Bedeutung sein.

So ist in einer bevorzugten Ausgestaltung weiter vorgesehen, dass die Umlenkstelle innenseitig eine abgeschrägte Fläche ausbildet und die der durchsichtigen Vorrichtungswand zugekehrte Umlenkstelle demgegenüber kantiger gestaltet ist. Zufolge dieser Ausgestaltung ist eine insgesamt weiche 180°-Umlenkung erreicht. Die Beanspruchung des über die Umlenkkante mitgeschleppten Streifenbandes ist gegenüber einer harten 180°-Umlenkung wesentlich verringert. Hierzu ist insbesondere die von der durchsichtigen Vorrichtungswand abweisende Umlenkstelle der Umlenkkante abgeschrägt, so dass sich hieraus anstelle einer harten 90°-Umlenkung eine unterteilte Umlenkung von bevorzugt zweimal 45° erreichen lässt. Zur eindeutigen Anzeige des Füllstand-Ist-Zustandes der Vorratskammer ist die der durchsichtigen Vorrichtungswand zugewandte Umlenkstelle im Vergleich zu der nach innen gewandten Umlenkstelle kantiger gestaltet, weiter beispielsweise eine unmittelbare 90°-Umlenkung anbietend, wobei weiter bevorzugt zur Materialschonung des Streifenbandes auch diese Umlenkstelle im Querschnitt verrundet oder abgeschrägt verlaufend ausgebildet sein kann. Da der, der durchsichtigen Vorrichtungswand zugewandte Umlenkstelle zugeordnete Streifenbandabschnitt den Anzeigewert darstellt, ist im Beispielsfall einer entsprechenden Verrundung der Umlenkstelle ein Radius im Zehntel-Millimeter-Bereich gewählt, so dass entsprechend die Eindeutigkeit der Anzeige gegeben ist.

Nachstehend ist die Erfindung anhand der beigefügten Zeichnung, welche lediglich ein Ausführungsbeispiel darstellt, näher erläutert. Es zeigt:
- Fig. 1: die Dosiervorrichtung in einer partiell geschnittenen Ansicht, die Grundstellung der Dosiervorrichtung vor einer Erstbenutzung betreffend;

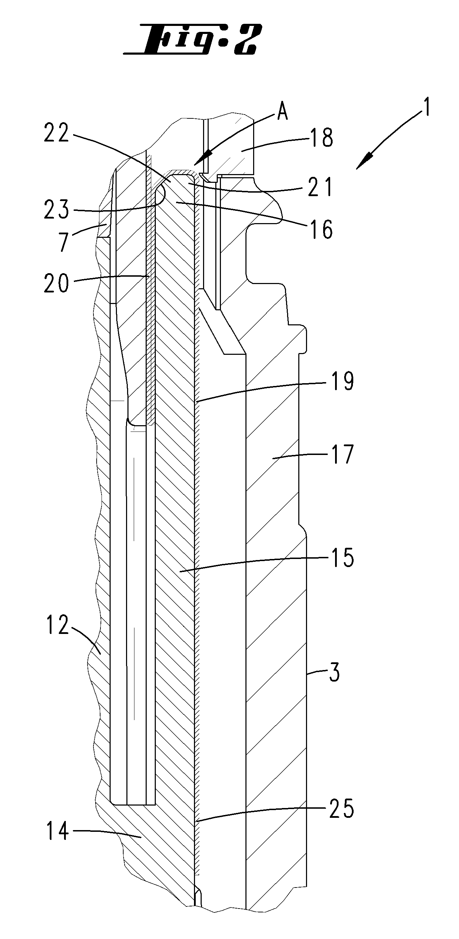

- Fig. 2: die Herausvergrößerung des Bereiches II in Fig. 1;

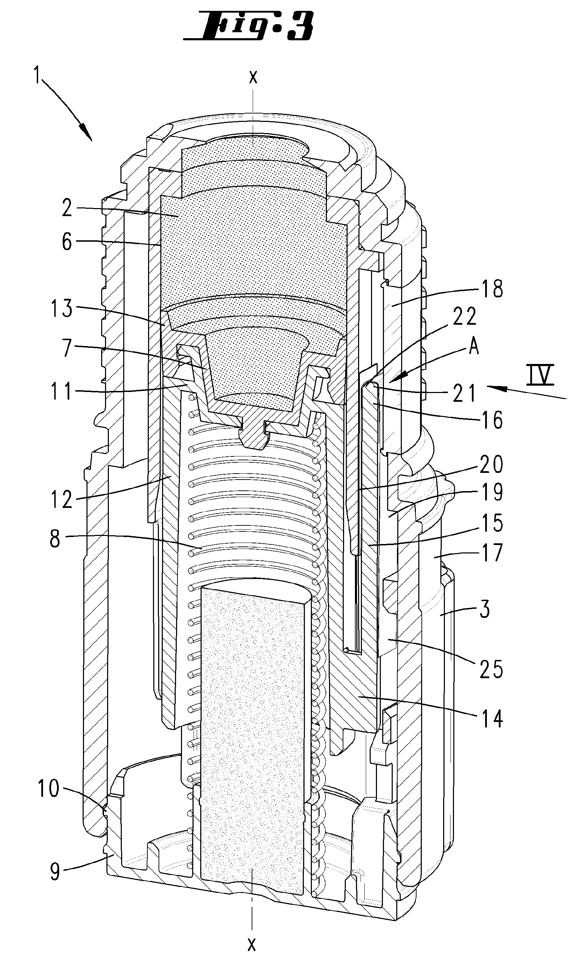

- Fig. 3: in perspektivischer Schnittdarstellung den in Fig. 1 geschnittenen Bereich der Dosiervorrichtung eine Zwischenstellung betreffend;

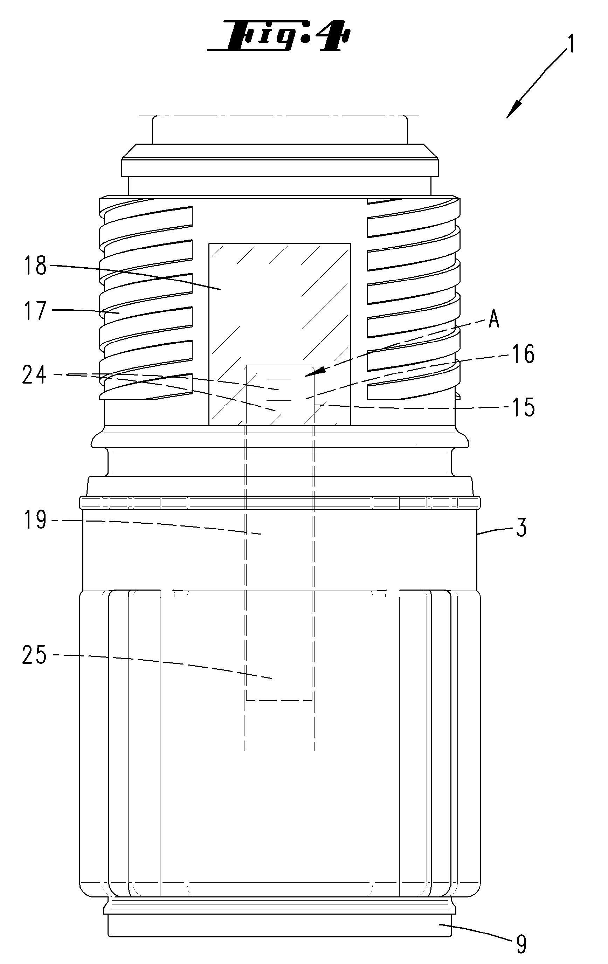

- Fig. 4: die Ansicht gegen den Dosiervorrichtungsbereich gemäß dem Pfeil IV in Fig. 3;

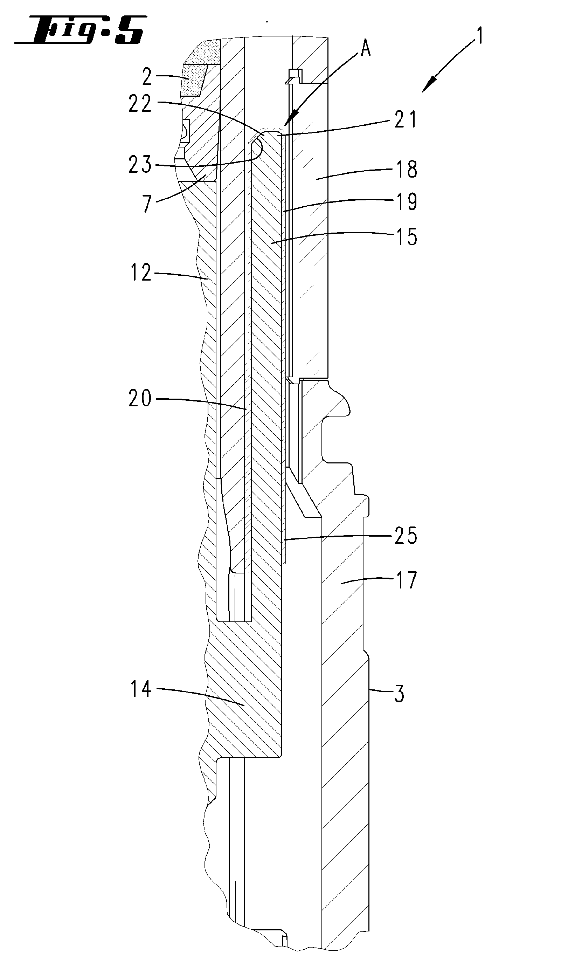

- Fig. 5: eine der Fig. 2 entsprechende Detail-Schnittdarstellung, jedoch eine weitere Zwischenstellung nach Durchführung mehrerer Inhalationsvorgänge und entsprechender Substanzentnahme aus der Vorratskammer betreffend;

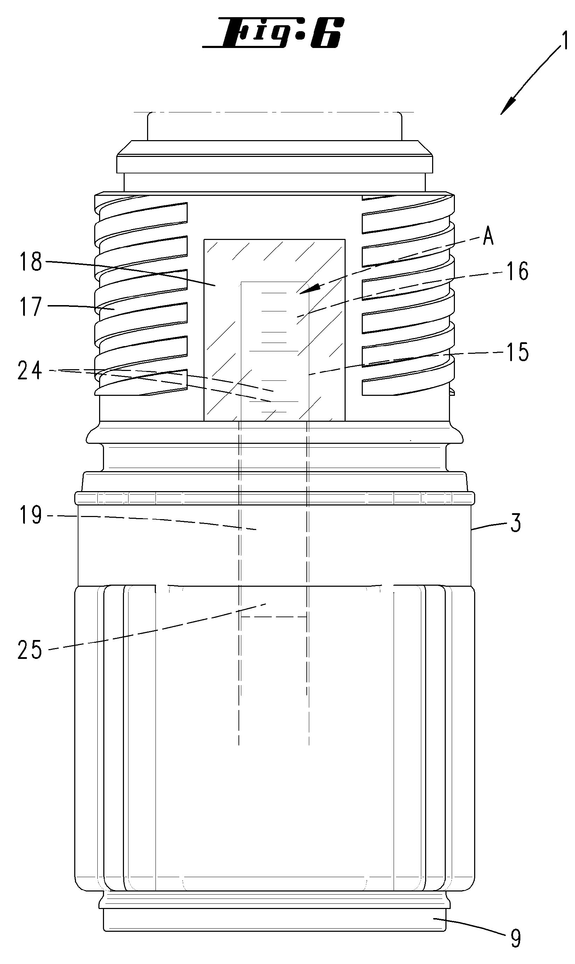

- Fig. 6: eine der Fig. 4 entsprechende Darstellung, jedoch die Stellung gemäß Fig. 5 betreffend.

Die in den Figuren dargestellte Dosiervorrichtung 1 zur Inhalierung einer pulverförmigen Substanz 2, insbesondere medizinischer Art, ist als bequem mitführbares, kurzstabförmiges Taschengerät realisiert, dies mit einem formbestimmenden zylindrischen Gehäuse 3.

Das zylindrische rohrartige Gehäuse 3 besitzt kopfseitig einen Außenzylinder, dessen freies Ende ein Mundstück 5 ausformt. Letzteres ist mundgerecht ausgebildet, beispielsweise abgeflacht. Das Mundstück 5 ist mittels einer nicht dargestellten, becherförmigen Verschlusskappe schützend überfangbar.

Die Substanz 2 ist in einer Vorratskammer 6 des Gehäuses 3 (gegebenenfalls nachfüllbar) aufgenommen. Über eine nicht dargestellte Dosiervorrichtung wird vorbereitend für einen Inhalationsvorgang eine Substanz-Teilmenge an eine außerhalb der Vorratskammer 6 liegende Übergangsstelle gefördert, entsprechend in eine Entleerungsbereitschaftsstellung verbracht.

Bezüglich des dosierfähigen Gutes handelt es sich um eine (meist medizinische) pulverförmige Substanz 2. Es können beispielsweise saugstromtransportfähige Grundkörper wie Laktoseträger für oberflächenseitig anhaftende, mikronisierte Arzneimittel-Feinpartikel sein.

Den unteren Abschluss der Vorratskammer 6 bildet ein topfförmiger Boden 7, welcher in Richtung des Mundstückes 5 mittels einer Druckfeder 8 unter Federbelastung steht. Die Druckfeder 8 stützt sich mit der fußseitigen Endwindung an einer das Gehäuse 3 dort schließenden Bodenkappe 9 ab. Diese steht in Rasteingriff zum hier wandungsinnenseitig querschnittsvergrößerten Abschnitt des Gehäuses 3, wobei ein entsprechender Rastkragen 10 der Bodenkappe 9 in eine passende Ringnut des Gehäuses 3 eingreift.

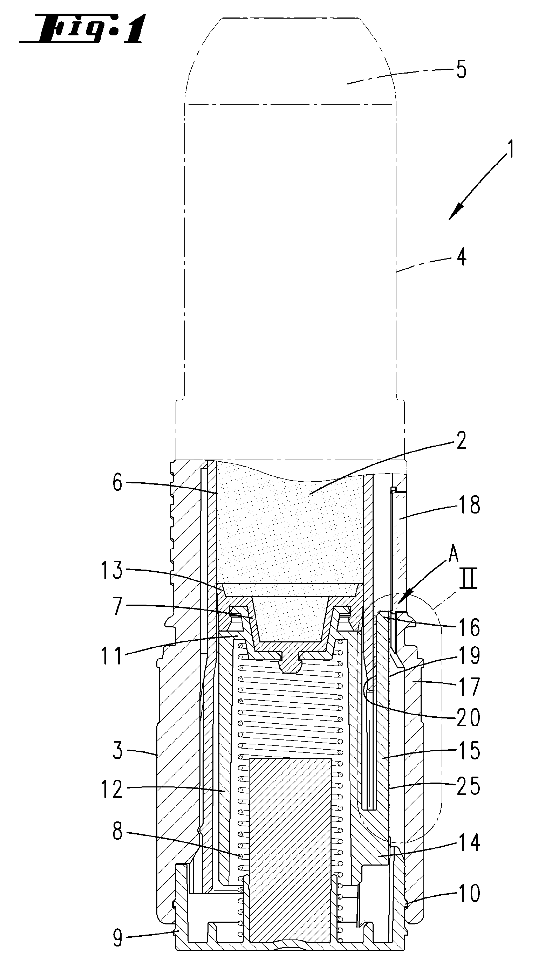

Die kopfseitige Endwindung der vorgespannten Druckfeder 8 wirkt belastend auf eine Innenschulter 11 eines Hohlkolbens 12 der kolbenförmigen Einrichtung 7/12. Wie insbesondere aus der Darstellung in Fig. 1 zu erkennen, ist der gestuft topfförmige Boden 7 im Bereich der Innenschulter 11 mit dem Hohlkolben 12 rastverbunden.

Der Topfrand des Bodens 7 stellt eine Ringlippe 13, die aufgrund ihres gummielastischen Materials die Wandung der Vorratskammer 6 verlustfrei abstreift.

Die Druckfeder 8 ist in dem dargestellten Ausführungsbeispiel eine Zylinderfeder, mit einer im entspanntem Zustand gemessenen Länge, die etwa einem Zehnfachen der maximalen Anpresslänge entspricht. Die Anpresslänge ist definiert durch das Axialverlagerungsmaß des Bodens 7 zwischen einer unteren, der Befüllstellung entsprechenden Stellung gemäß Fig. 1 und einer oberen, anschlagbegrenzten Stellung des Bodens 17 in der Vorratskammer 6. So ist in dem dargestellten Ausführungsbeispiel eine solche Anpresslänge von 15 mm gegeben. Zufolge der Federausgestaltung, insbesondere zufolge der gewählten Federlänge ist über die gesamte Anpresslänge ein konstanter Federdruck auf den Boden 7 erreicht, was zu einer gleichmäßigen Substanzverdichtung über den gesamten Nutzungszeitraum der Vorrichtung 1 führt.

Der mit dem Boden 7 rastverbundene Hohlkolben 12 weist fußseitig einen Radialausleger 14 auf. An diesem ist ein, die Vorratskammerwandung wandungsaußenseitig übergreifender, axial in Richtung auf die Ebene des Bodens 7 ausgerichteter, fingerartiger Vorsprung 15 angeformt. Durch die Anbindung des Vorsprunges 15 an den Hohlkolben 12 ist die axiale Stellung des Vorsprunges 15, insbesondere die axiale Stellung des freien Endes 16 des Vorsprunges 15 direkt abhängig von der axialen Stellung des Bodens 7.

Dem freien Ende 16 des Vorsprunges 15 zugeordnet ist die Vorrichtungswand 17 des Gehäuses 3 transparent gestaltet, formt entsprechend ein Sichtfenster 18 aus. Die in Axialerstreckung der Dosiervorrichtung 1 betrachtete Höhe des Sichtfensters 18 ist angepasst an das maximale Axialverlagerungsmaß des Bodens 7 in der Vorratskammer 6 zwischen dessen unteren Stellung und der oberen, anschlagbegrenzten Stellung. Diese obere anschlagbegrenzte Stellung des Bodens 7 entspricht der Entleerungsstellung der Vorratskammer 6, d. h. der Stellung, aus welcher keine weitere Inhalation mehr heraus erfolgen kann, wenngleich durchaus ein nicht mehr austragbarer Substanzrest in der Vorratskammer 6 verbleiben kann.

Der Vorsprung 15 wirkt mit einem Streifenband 19 zusammen. Dieses ist im Bereich eines freien Endes im Überdeckungsbereich von Vorsprung 15 und Vorratskammerwandung an Letzterer angebunden, beispielsweise bei einer Ausgestaltung des Streifenbandes 19 aus einem Kunststoffmaterial an der Vorratskammerwandung verklebt oder verschweißt.

Ausgehend von dieser Bandanbindung 20 erstreckt sich das Streifenband 19 zunächst entlang der der Vorratskammerwand zugewandten Fläche des Vorsprunges 15. Nach einer 180°-Umlenkung um das freie Ende 16 des Vorsprunges 15 verläuft das Streifenband 19 entlang der nach radial außen gerichteten Fläche des Vorsprunges 15 nach unten in Richtung auf den Bodenabschnitt der Dosierrichtung 1. Auf den jeweils zugewandten Flächen des Vorsprunges 15 liegt das Streifenband 19 zur Schiebeverlagerung desselben lose auf.

Zufolge dieser Anordnung ist eine Anzeige A erreicht, zur visuellen Übermittlung des Füllstandgrades der Vorratskammer 6. Die aus der Axialverlagerung des Bodens 7 resultierende, direkte Bewegung des Vorsprunges 15 wird zufolge des einendig an einem gehäusefesten und somit nicht axial beweglichen Bereich festgelegten und lose um 180° um den Vorsprung 15 gelegten Steifenbandes 19 auf dieses indirekt übertragen. Eine Axialbewegung des Vorsprunges 15 zufolge einer Verlagerung des Bodens 7 nach einer Teilsubstanzentnahme aus der Vorratskammer 6 wird entsprechend dem Flaschenzug-Prinzip auf das Streifenband 19 übertragen, so dass ein Übersetzungsverhältnis von Vorsprungs-Axialverlagerungsmaß zu Streifenband-Axialverlagerungsmaß im Bereich des auf der Außenseite des Vorsprunges 15 frei aufliegenden Abschnittes von 1:2 erreicht ist.

Im Zuge der sukzessiven Axialverlagerung des Vorsprunges 15 in Abhängigkeit von der nach und nach durchgeführten Teilsubstanzentnahme aus der Vorratskammer 6 wandert das freie Ende des Vorsprunges 15 sichtbar hinter dem Sichtfenster 18 nach oben, dies unter entsprechender übersetzender Mitschleppung des Streifenbandes 19, wobei die dem Sichtfenster 18 zugewandte Umlenkstelle 21 des Vorsprungs 15 den Anzeigewert auf dem Streifenband 19 definiert. In dem dargestellten Ausführungsbeispiel ist diese Umlenkstelle 21 im Querschnitt relativ scharfkantig gestaltet, lediglich mit einer Verrundung versehen, deren Radius im Zehntel Millimeterbereich liegt. So ist entsprechend eine kantige Umlenkstelle 21 geformt, wodurch eine eindeutige Ablesbarkeit der Anzeige A erreicht ist.

Die nach innen gewandte, d. h. der äußeren Umlenkstelle 21 abgewandte Umlenkstelle 22 ist gegenüber der Umlenkstelle 21 entschärft, dies durch Ausformung einer abgeschrägten Fläche 23. Zufolge dieser Ausgestaltung wird das über das freie Ende 16 des Vorsprunges 15 geführte Streifenband 19 innenseitig in dem dargestellten Ausführungsbeispiel über zwei in Streifenbandlängserstreckung hintereinander folgende 45°-Umlenkungsbereiche geführt. Dies erweist sich als materialschonend für das Streifenband 19. In alternativer Ausgestaltung kann die Umlenkstelle 22 gleich der äußeren Umlenkstelle 21 im Querschnitt verrundet sein, dies weiter bevorzugt mit einem gegenüber der Umlenkstelle 21 wesentlich vergrößerten Radius.

Wie in den Figuren dargestellt, ist das Streifenband 19 sichtseitig, d. h. mit der im Bereich der Umlenkstelle 21 dem Sichtfenster 18 zugewandten Seite mit einer Skalierung 24 versehen. Diese zeigt die Anzahl der noch durchführbaren Inhalationen, indirekt die Anzahl der noch aus der Vorratskammer 6 abzuteilenden Substenzmengen an. Alternativ kann über die Anzeige A bzw. über die Skalierung 24 auch die Anzahl der bereits durchgeführten Inhalationen dargestellt werden.

Weiter alternativ ist sichtseitig das Streifenband 19 mit einem Farbverlauf versehen. So ist beispielsweise das lose freie Ende 25 des Streifenbandes 19, welches kurz vor Anschlagen des Bodens 7 in der obersten Stellung in den Sichtfensterbereich sowie weiter in den Bereich der Umlenkstelle 21 gelangt mit einer Warnfarbe, beispielsweise rot, versehen, während der restliche Sichtbereich des Streifenbandes 19 eine demgegenüber neutrale Farbe, wie beispielsweise gelb aufweist.

Alle offenbarten Merkmale sind (für sich) erfindungswesentlich. In die Offenbarung der Anmeldung wird hiermit auch der Offenbarungsinhalt der zugehörigen/beigefügten Prioritätsunterlagen (Abschrift der Voranmeldung) vollinhaltlich mit einbezogen, auch zu dem Zweck, Merkmale dieser Unterlagen in Ansprüche vorliegender Anmeldung mit aufzunehmen.

### BEZUGSZEICHENLISTE

- 1: Dosiervorrichtung
- 2: Substanz
- 3: Gehäuse
- 4: Außenzylinder
- 5: Mundstück
- 6: Vorratskammer
- 7: Boden
- 8: Druckfeder
- 9: Bodenkappe
- 10: Rastkragen
- 11: Innenschulter
- 12: Hohlkolben
- 13: Ringlippe
- 14: Radialausleger
- 15: Vorsprung
- 16: freies Ende
- 17: Vorrichtungswand
- 18: Sichtfenster
- 19: Streifenband
- 20: Bandanbindung
- 21: Umlenkstelle
- 22: Umlenkstelle
- 23: Fläche
- 24: Skalierung
- 25: freies Ende

- A: Anzeige

## Patentansprüche

1. Dosiervorrichtung (1) zur Inhalierung einer pulverförmigen Substanz (2), insbesondere medizinischer Art, die in einer Vorratskammer (6) oberhalb eines nachlaufenden Bodens (7) angeordnet und aus dieser in eine Entleerungsbereitschaftsstellung bringbar ist, und mit einer dem echten Füllstand zugeordneten Anzeige (A) im Bereich der Vorrichtungswand (17), **dadurch gekennzeichnet, dass** die Anzeige (A) aus einem vom Boden mitgeschleppten, um 180° umgelenkten Streifenband (19) besteht, wobei der Umlenkbereich des Streifenbandes (19) hinter der durchsichtigen Vorrichtungswand (17) liegt.

2. Dosiervorrichtung nach Anspruch 1, **dadurch gekennzeichnet, dass** eine erste Umlenkstelle (22) innenseitig eine abgeschrägte Fläche (23) ausbildet und eine der durchsichtigen Vorrichtungswand (17) zugekehrte zweite Umlenkstelle (21) demgegenüber kantiger gestaltet ist.

## Claims

1. Dosing device (1) for inhaling a powdery substance (2), particularly of a medical nature, which is arranged in a storage chamber (6) above a trailing bottom (7) and can be brought from said storage chamber into an emptying readiness position, and having a display (A) associated with the actual filling level in the area of the device wall (17), **characterized in that** the display (A) is composed of a strip tape (19) carried along by the bottom and deflected by 180°, wherein the deflection area of the strip tape (19) lies behind the transparent device wall (17).

2. Dosing device according to Claim 1, **characterized in that** a first deflection point (22) forms a beveled surface (23) on the inner side and a second deflection point (21) facing the transparent device wall (17) is configured more edged by comparison.

## Revendications

1. Dispositif de dosage (1) pour l'inhalation d'une substance pulvérulente (2), en particulier médicale, qui est disposée dans une chambre de réserve (6) au-dessus d'un fond arrière (7) et qui peut être amenée hors de celle-ci dans une position prête à être distribuée, et comprenant une indication (A) associée au niveau de remplissage actuel dans la région de la paroi du dispositif (17), **caractérisé en ce que** l'indication (A) se compose d'une bande de ruban (19) entraînée avec le fond, déviée de 180°, la région de déviation de la bande de ruban (19) étant située derrière la paroi transparente du dispositif (17).

2. Dispositif de dosage selon la revendication 1, **caractérisé en ce qu'**un premier point de déviation (22) constitue, du côté intérieur, une surface biseautée (23) et un deuxième point de déviation (21) tourné vers la paroi transparente du dispositif (17) est configuré sous forme plus anguleuse par rapport à celui-ci.
